# EUROPEAN PATENT APPLICATION

(11) **EP 1 875 934 A1**
(43) Date of publication of application: **09.01.2008**
(21) Application number: 06116715.1
(22) Date of filing: 06.07.2006
(51) Int. Cl.: A61M 5/19, A61M 5/315

(54) **Prefilled medical expelling device, dual chamber**

(71) Applicant: NOVO NORDISK A/S, 2880 Bagsvaerd (DK)
(72) Inventor: The designation of the inventor has not yet been filed

(57) **Abstract**

A drug expelling medical device has a drug storage chamber (1) and a drug pressure chamber (2). The drug contained in the storage chamber is transferred via a transfer channel (9) to the pressure chamber by moving a slidable plunger (5) forming a liquid barrier between the two chambers in a first direction, whereby the volume of the storage chamber is decreased and the volume of the pressure chamber is correspondingly increased. The plunger is sealed towards the chamber wall(s) and an optional orifice in the pressure chamber can be sealed off prior to the drug expelling.

## Description

The invention relates to a prefilled medical drug expelling device, in one embodiment a jet injection device equipped with a drug storing chamber and a drug pressure chamber. The device is constructed to enable long time storing, sterilization and high pressure expelling though all accomplished in a simple, low cost design. The invention also relates to a method of transferring a liquid drug from a first storing chamber to a second pressure chamber in a simple manner.

### BACKGROUND OF THE INVENTION

Subcutaneous and intramuscular delivery of liquid drugs by injection is common in the medical arts. As some medications such as insulin must be given frequently by injection to an individual, easy performance of the injections is desirable.

Many patients dislike needle injections due to pain or fear for needles. Further, blood-borne pathogens, such as HIV and hepatitis, can be transmitted to health care workers by accidental needle-sticks. Also, the disposal of used needles is a growing concern. This disposal presents a problem to individuals other than healthcare workers. Children, for example, may find used needles in the garbage, putting them at risk of contracting infection. Discarded needles likewise pose a risk to waste disposal workers. This is at the moment a huge worldwide problem,

In efforts to minimize the fears and risks associated with needle injections, several types of needle-free jet injectors have been developed. These devices penetrate the skin using a high velocity fluid jet and deliver medication into the tissue of a patient. In order to accomplish this, a force is exerted on the liquid medication. Jet injectors in general contain a fluid drug which has been transferred into a chamber having a small orifice at one end. A drive means, e.g. a ram, is accelerated using either a coil spring or a compressed gas energy source. The ram impacts a plunger which in turn creates a high variable pressure profile within the chamber. This pressure ejects the fluid medicament through the orifice at high velocity, piercing the skin. The energy source continues to apply a force to the plunger which quickly propels the drug through the opening in the skin, emptying the injection chamber in a fraction of a second. The drive means may be adapted to provide a two-stage injection, i.e. a first penetrating burst of drug at a high pressure followed by a subsequent delivery of the remaining amount of drug at a lower pressure. It shall be noted that the same principle of having a pressurized injection device can also be used for needle-injectors, in the case where it is desirable to enable an injection without a person having to press in the drug by her- or him-self when injecting. This can be the case if it is desirable to have a well known injection pressure and time, or if the user feels discomfort by having to perform the actual injection.

In order to ensure ease of use and user safety, it is often desirable to deliver medical devices as single-use units. Single-use devices often requires less handling by the user which is off course easier, but more important safer for the patient who in many cases has to perform the handling of the device by her- / him-self. The less handling the smaller risk of performing a wrong and potential hazardous act. To further ease the handling and minimize the risk of performing a faulty act it is desirable that the medical device is prefilled. When however, the medical device is a pressure device, the problem is to provide a prefilled device that is both stable, pressure resistant, simple and inexpensive.

A device made of a plastic material can be manufactured quick and with low cost, assuming there are not many components to be produced and assembled, but as regulations for medical devices demand for the device containing a liquid drug to be transparent, it can be difficult to also meet the requirements of stability over more than a 2 year period. Plastic materials are not entirely impenetrable and stable. Therefore oxygen can enter through the containing plastic material and into the drug, molecules from the drug can escape out through the plastic material and still another problem is that unwanted material from the drug containing plastic material can be released from the container and mix with the liquid drug, potentially causing side effects to the patient.

Many of these drawbacks can be overcome by using glass as the containing material, but then the problem arises concerning pressure resistance. Hardened glass may provide the necessary pressure resistance when produced in sufficient dimensions, but this renders the device relative expensive for single-time use.

A solution to the problems described is presented in WO 01/89614. Here a high pressure medical device is equipped with a storing chamber bordered by glass and two resilient plungers, enabling for long time storage. Prior to an injection, the liquid drug is transferred from this stable storing chamber to a pressure resistant chamber by moving the two plungers towards the pressure chamber and relatively closer to each other. However this solution still suffers from some unfortunate drawbacks. There is a three step procedure described, transfer/air-removing/injection, which could be reduced and also, as all these three steps are done by moving the plunger in one and the same direction, it requires a relative long device to accommodate these functions. Also, WO 01/89614 has plungers made from a resilient material. When performing a high pressure injection, the pressure on the plungers will force the plunger material radially outwards, which means the friction loss between the plunger and the surrounding wall will be huge at high pressures. Further the resilient material makes it difficult to acquire a well defined force profile during an injection: If a peak pressure is desired in the first stage of an injection followed by a low pressure, the resilient material will dampen the force profile and cause fluctuations.

### PROBLEM TO BE SOLVED

It is the objective of the present invention to achieve a prefilled medical device which is easy to manufacture and easy in use, and which has relative low manufacturing costs. The device must allow for stable long time storage, even when prefilled with a medical drug, and it shall be possible to sterilize and keep sterilized during production, storage and end use.

In view of the above, it is a further objective of the present invention to achieve a medical device with the mentioned features, but where the mentioned draw-backs regarding dimensions, handling steps, friction loss and injection force profile, from the known related art is overcome.

An alternative aim of the present invention is to provide a pressurized injection device with resemblance of a conventional pen type injector as regards function and configuration, in order to make the patient comfortable with the jet injection device and so that the injection device can easily be utilized by a non-professional user, e.g. a insulin requiring diabetic.

### SOLUTION TO THE PROBLEM

The solution to the above mentioned problems is a medical device according to the independent claims with specified more detailed embodiments according to the dependant claims. The features according to the claims are further elaborated in the following:

### SUMMARY OF THE INVENTION

In the disclosure of the present invention, embodiments and aspects will be described which will address one or more of the above objectives or which will address objectives apparent from the below disclosure as well as from the description of exemplary embodiments.

In a first aspect, a medical drug expelling device is provided comprising a first chamber for holding a drug during storage for long time (approximately 2 - 3 years), and a second chamber for holding the drug immediately prior to the actual expelling of the drug. The medical device can be equipped with means for injecting the drug in its distal end. Particularly when the medical device is a jet injection device for injecting a drug under high pressure, the device can have an orifice in liquid connection to the second chamber, the second chamber then being a pressure chamber. Though a jet injector with high pressure is able to penetrate the skin and create a passage during the first phase of an injection, it is also known to combine the jet injector with a small needle, which will then penetrate the skin just prior to the injection.

The mentioned two drug chambers can be of primarily cylindrical shape, having mainly the same axis. The chambers can be made of different materials serving for different purposes. As one chamber is intended for long time storage of a medical drug, it is of high concern that this chamber is made from a stable and dense material, which further is transparent; in order to minimize the risk of contaminating the drug either with foreign matter from outside the medical device or with foreign matter from the actual chamber material itself. The other chamber does not serve for storing, but for delivering the drug to an injection, and the demands are therefore more to strength, in case of a high pressure injection, and not so much to long term stability and density, but still it is a demand that the material is transparent as it is containing a drug to be injected. Apart from transparency, an overall demand for a potential on-time-use medical device is off course a low material and production price. Considering these demands, useful materials are various glass types for chamber one, the storing chamber, and various plastic types for chamber two, the pressure chamber.

During production, these two chambers are assembled by known assembling methods, which are not the issue of the present invention, with mainly the same axis as mentioned. Located inside the chamber, and forming the barrier dividing the two chambers to two separated volumes, is a slidable plunger. It is not a demand that the plunger is of a transparent material, and therefore more material choices are available when meeting the conflicting demands dealing with the question of both storing and pressure, which is the case for the plunger as it is located in the zone exposed to both the storing chamber and the pressure chamber. It is however important that the plunger is made of a non-resilient material, to ensure a low friction loss when sliding during a high pressure injection, as well as a well defined pressure profile throughout the injection, which is not achievable it the plunger is resilient and therefore deforms and takes up energy in the material when submitted to a high pressure. A possibility is to make the plunger in two different materials, one material exposed to the pressure chamber, the other material exposed to the storage chamber. Like the two chambers, it is then possible to make the material of the plunger suit the given demands, especially the demands relating to stability and density versus the demands relating to strength and incompressibility. To ensure enclosure of the drug in the storing chamber, one or more sealing can be provided in the space between the drug chamber walls and the plunger. This sealing can be of a resilient type, but as they are of a very small area relative to the total cross-sectional area of the plunger, their deformation and friction exerted when sliding along the chamber wall(s), when submitted to high pressure during an injection, is insignificant.

To enable transfer of a drug stored in the storing chamber to the pressure chamber, one or more transfer passages are located between the two chambers (in the following, the one or more passages will be referred to in the singular for simplicity). The transfer passage enable the drug to by-pass the plunger otherwise forming a sealed-off border between the chambers. A variety of locations of the passage is possible: It can pass from a penetration in the storing chamber wall, further outside the chambers and back through a penetration in the pressure chamber wall. It can pass via a channel formed on the inside of the chamber wall surfaces. It can be enabled via the construction of the seal between the plunger and the chamber walls, whereby the seal functions as a check valve. It can pass as a channel inside the plunger.

Whatever construction of the passage is chosen, it is important to ensure that no drug can pass from the storing chamber to the pressure chamber or foreign matter the opposite way unintentionally to ensure stability and purity of the drug. In the case where an orifice is in liquid connection to the pressure chamber, there is a risk of contamination via the orifice to the chambers. A solution is to equip the orifice and the area around it intended to be held against the skin of a subject during an injection with a closure integrity member. The closure integrity member can be in the form of a plug blocking and sealing the orifice potentially also combined with a peelable folio or a cap covering the area around the orifice. In this case, where the orifice is sealed off, the only other opening to the pressure chamber is the passage to the storing chamber. Therefore, if this passage is a channel of small cross-sectional area and therefore has a high (longitude) / (cross-sectional area) ratio, no drug will pass from the storing chamber to the pressure chamber, and likewise, no foreign matter will pass from the pressure chamber to the storing chamber, so no further sealing is needed. As a further security though, optionally also the channel can be blocked by a sealing, potentially a plug fixed to the inside pressure chamber wall oppositely the channel, thereby blocking the channel when the plunger is in its extreme proximal position.

As the plunger is positioned between the two chambers, retracting the plunger from its initial extreme proximal position, which is the position in which it is delivered to the user from production, in a first direction to its distal position, will reduce the volume of the first chamber (the drug storing chamber) and at the same time will increase the volume of the second chamber (the drug pressure chamber). As the liquid drug is incompressible, it will be forced through the passage from the first chamber and transferred to the second chamber, this being the loading sequence of the medical device. It is possible to transfer all the drug or only part of the drug, as the medical device according to this invention can be used both as a prefilled single-use device with a fixed dose size or as a multi-dose device with the possibility of dialing up a user defined dose. The storage chamber outer limits are defined by the chamber wall, the distal side of the plunger inclusive seal and a ram shaft and a proximal end wall including a seal towards the ram shaft. This end wall does not need to be transparent and neither does it have to be rigid. Therefore there is a range of possible material choices wider than those of the chamber wall and the plunger. Actually the end wall can be made of a resilient material and possibly therefore forming the seal itself against the chamber wall and the ram shaft, without a further additional seal provided. If the device is designed for delivering more than one dose, it can be advantageous to have a movable end wall in the storage chamber, which then every time a dose is dialed and injected will advance in a proximal direction, stepwise minimizing the volume of the storage chamber according to the amount of drug used.

The loading sequence can be a combination of two activities: The loading of the drug from the storing chamber to the pressure chamber, and the loading of a force element adapted to supply and transfer the necessary force profile to the plunger. A variety of force elements and transfer principles are well known in the art and will not be discussed here, as this is not the scope of the present invention. As these two loadings can be performed simultaneously, it is only necessary to take off an optional closure integrity member, and the medical device is now ready to perform an injection. This means the medical device has a very easy handling consisting of only two handling steps, the loading step and the injection step. The injection step consists only of placing the medical device against the skin of a body on the desired location and pressing the injection button, optionally after releasing a safety mechanism. The force element will then advance the plunger in a second proximal direction from the loaded distal position to its extreme proximal position. This will force the drug from the pressure chamber out of the orifice and in the case where the medical device is held against a skin surface, the liquid jet then formed will penetrate the skin and an injection is performed. In order to reduce or totally avoid a back-flow of the drug from the pressure chamber back to the storage chamber, the liquid transfer channel through the plunger can have a small cross-sectional area, an intricate shape or it can comprise a check valve to totally block the channel against flow in the first distal direction. In the case mentioned, where the plunger is made of two materials, one suitable for the different conditions in the storage- and the pressure chamber, it is a possibility that the plunger side exposed to the storage chamber is made by a resilient material. This gives the opportunity to make a seal towards the chamber wall as well as a check valve in the transfer channel an integrated part of the resilient plunger part. As the proximal shape of the plunger is carefully fitting the inner proximal end part of the pressure chamber, the amount of drug remaining in the pressure chamber after the injection step is performed is exiguous.

Depending whether the medical device is for one- or multi time use, and whether it is a complete device or connected to a multi-use force-unit, the medical device can now be discarded or protected and stored for further use. One of the advantages is that in case it is a needle free device, there is not the great potential hazard of spreading diseases as is a big problem in many environments when dealing with needle devices.

### DESCRIPTION OF THE DRAWINGS

In the following the invention will be further described with references to the drawings, wherein
- Fig. 1: shows a conventional principle of loading a medical device.
- Fig. 2: shows a pressurized injection device according to the invention in three different situations.

### POSITION NUMBERS OF THE DRAWINGS

- 1:: Drug Storage Chamber, First Chamber
- 2:: Drug Pressure Chamber, Second Chamber
- 3:: First Chamber Wall (Drug Storage Chamber)
- 4:: Second chamber Wall (Drug Pressure Chamber)
- 5:: Slidable Plunger
- 6:: Orifice / (needle)
- 7:: Distal End Wall of Storage Chamber
- 8:: Ram Shaft of the Slidable Plunger
- 9:: Transfer Passage
- 10:: Plunger Sealing
- 11:: Ram Shaft Sealing
- 12:: Transfer Passage Sealing

- 20:: Adapter
- 21:: Transfer Needle
- 22:: Gripping Edge

- 30:: Drug Cartridge
- 31:: Pierceable Septum

### DESCRIPTION OF EXEMPLARY EMBODIMENTS

When in the following terms as "distal", "proximal" and "radial" or similar relative expressions are used, these only refer to the appended figures and not necessarily to an actual situation of use. The shown figures are schematic representations for which reason the configuration of the different structures as well as there relative dimensions are intended to serve illustrative purposes only.

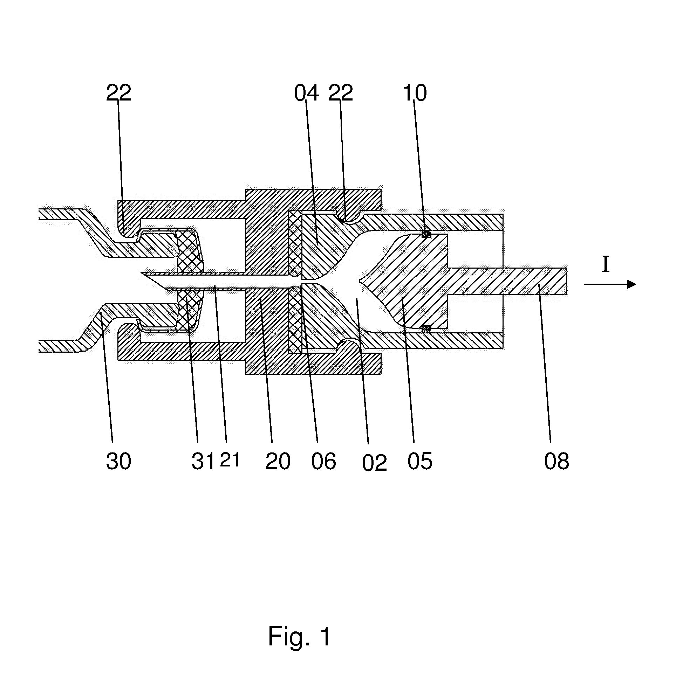

Figure 1 depicts a prior art method and device for loading a liquid drug into a medical drug expelling device. An adaptor 20 is fixed to the outside of the drug pressure chamber via one of the gripping edges 22 on the adaptor. Likewise a liquid drug containing cartridge 30 is fixed to the other end of the adaptor via the other of the adaptors gripping edges. In this way liquid passage is established between the pressure chamber 2 and the liquid drug contained in the cartridge via the hollow transfer needle 21 which pierces the septum 31 when the adaptor is fixed to the cartridge. The setup of adaptor, medical expelling device and cartridge is now ready for transfer of a dose of drug from the cartridge to the pressure chamber. This is performed by moving the slidable plunger 5 in a first direction (I) distal to the orifice 6 end of the expelling device. The space between plunger and the inside pressure chamber wall is sealed 10, therefore moving the plunger in a first distal direction and thereby expanding the pressure chamber volume, will create a lower pressure in the pressure chamber than inside the cartridge. As the distal end of the cartridge is bordered by a slidable piston (not shown) able to vary the cartridge volume, this lower pressure in the pressure chamber will drive liquid drug from the cartridge, thorugh the hollow transfer needle and to the pressure chamber, thereby loading the medical drug expelling device with a dose of drug.

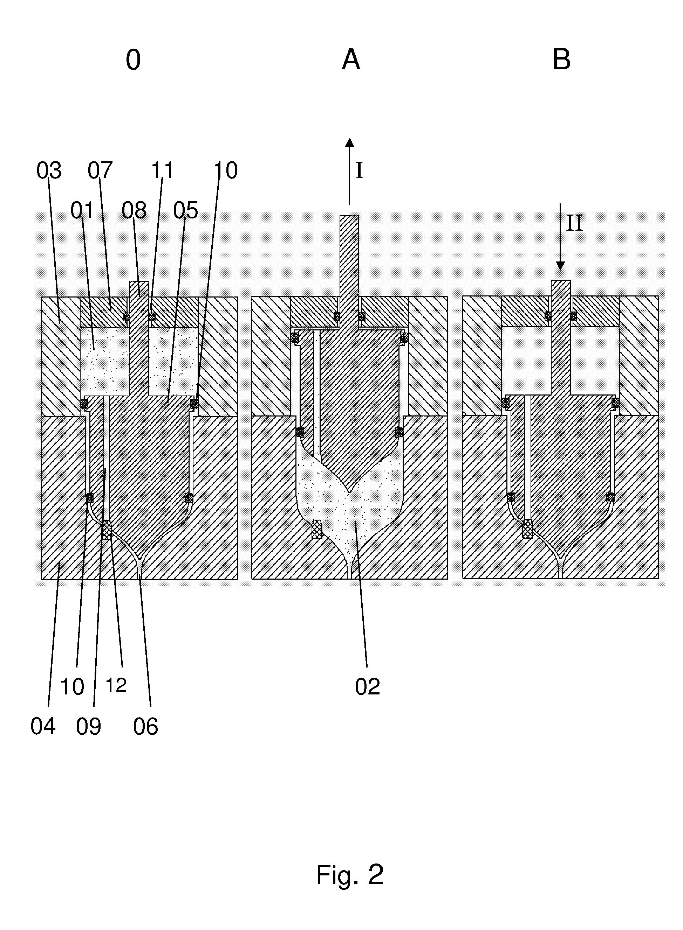

Figure 2 shows an alternative embodiment according to this invention, enabling a much simpler and easier loading procedure of medical drug expelling device. In this embodiment the medical drug expelling device is prefilled with a dose of ready-to-use liquid drug, this fact already simplifying the loading procedure. The medical device consists of two chambers, a drug storage chamber 1 and a drug pressure chamber 2. On figure 2, three situations are shown, 0, A and B. Situation 0 illustrates the medical device as it is delivered to the user, with a liquid drug in the storage chamber and the slidable plunger 5 in its extreme proximal position, whereby the volume of the pressure chamber is inconsiderable, since the shape of the proximal end of the plunger is intimate conformed to the proximal inside shape of the pressure chamber. The orifice 6 of the pressure chamber can be equipped with a closure integrity member (not shown as it is not the scope of this invention) to block and seal the orifice and also protect the surface of the medical device adapted to be put against the skin of a user on the location desired for an injection. This surface can also be provided with an adhesive or other means for retention of the skin. The storage chamber containing the drug is limited by the storage chamber wall 3, the distal side of the plunger including seal 10 and the proximal side of the distal end wall of the storage chamber 7 including seal 11 towards the ram shaft 8 of the plunger, as well as the ram shaft itself. Further, a limiting element of the storage chamber can be a transfer passage sealing 12, fixed to the proximal inside wall of the pressure chamber directly opposing the opening of the transfer passage 9 outlet. As can be seen on the figure 2, the diameter of the storage chamber is not exactly the same as the diameter of the pressure chamber. This is to compensate for the cross sectional area of the ram shaft, which reduces the volume of the storage chamber. By adjusting the storage chamber diameter and the pressure chamber diameter relative to one another, the volume of drug transferred from the storage chamber is carefully tallied to the volume of the pressure chamber. An alternative option is to adjust the cross sectional area of the storage chamber relative to the cross sectional area of the pressure chamber, so the effective volume of the storage chamber will be slightly greater than the effective volume of the pressure chamber, thereby incorporating the function of removing any excess air from the pressure chamber simultaneously to the transfer of drug from the storage chamber to the pressure chamber. Whatever relation between the two cross sections are chosen, it can be an advantage to have two sealing 10 as shown on figure 2.

Turning now to situation "A", the medical device is in its loaded stage. The slidable plunger has been moved in a first direction "I" to its extreme distal position. This has caused the liquid drug to be transferred to the drug pressure chamber through the transfer passage. As the seal 12 is fixed to the chamber wall, the transfer passage is open for passage when first the moving of the plunger has been initiated.

In situation "B" the medical device has expelled the liquid drug through the orifice. This has been done by applying a force profile to the ram shaft and further on to the slidable plunger. Thereby the liquid drug is forced out of the orifice when the plunger is moved in a second direction "II".

## Claims

1. A prefilled medical drug expelling device comprising;
a first (1) and a second (2) drug chamber, a nozzle comprising at least one expelling opening (6), a plunger (5) forming a barrier between the first and the second drug chamber, at least one passage (9) between the first and the second drug chamber, a prefilled drug being contained in the first chamber,
**characterized in that**, at least a rigid part of said plunger is in contact with the second drug chamber and that the plunger is adapted to be moved in a first (I) direction, whereby the drug is transferred from the first to the second drug chamber and a second (II) direction, whereby the same drug is expelled through the expelling opening(s).

2. A medical device according to claim 1,
**characterized in that**, said passage between the first and the second drug chamber is a channel through said plunger.

3. A medical device according to claim 1 or 2,
**characterized in that**, sealing means (12) are provided inside the second drug chamber, said sealing means are adapted to cooperate with the passage(s), thereby sealing the passage(s) when the plunger is moved to its extreme second position.

4. A medical device according to any of the preceding claims,
**characterized in that**, sealing means (10) are provided between the plunger and at least one of the chamber walls (3/4), thereby ensuring sealing between the first drug chamber and the second drug chamber.

5. A medical device according to any of the preceding claims,
**characterized in that**, said passage comprises flow restricting means to reduce reverse flow from the second drug chamber to the first drug chamber.

6. A medical device according to claim 5,
**characterized in that**, said flow restricting means is a check valve, a small cross-sectional area or an intricate shape.

7. A medical device according to any of the preceding claims,
**characterized in that** said plunger is made from two materials with different properties in at least one sense.

8. A medical device according to any of the preceding claims,
**characterized in that** the plunger material in contact with the first drug chamber forms a seal towards the chamber wall, and optionally integrates a check valve function to ensure one way flow in said passage(s)

9. A medical device according to any of the preceding claims,
**characterized in that**, said expelling opening(s) is at least one orifice, said orifice comprises closure integrity means sealing off the orifice and protecting the area around the orifice.

10. A medical device according to any of the preceding claims,
**characterized in that**, said first chamber is a storing chamber and said second chamber is a pressure chamber.

11. A medical device according to any of the preceding claims,
**characterized in that**, said two chambers are made from different materials, preferably said first chamber is made from glass and said second chamber is made from a transparent plastic.

12. A medical device according to any of the preceding claims,
**characterized in that**, when the slidable plunger is in its extreme second position the first chamber is of slightly greater volume than the volume of the second chamber when the slidable plunger is in its extreme first position, thereby enabling expelling of excess air in the second chamber simultaneously to the transfer of the liquid drug from the first chamber to the second chamber.

13. A medical device according to any of the preceding claims,
**characterized in that**, said medical device is a medical jet injection device.

14. A method for transferring a liquid drug from a first drug chamber being prefilled with a liquid drug, to a second drug chamber, and expelling said liquid drug from said second drug chamber through an expelling opening in a medical drug expelling device, comprising the steps of,
i. reversing an at least partly rigid plunger located between said first and said second drug chamber in a first direction, thereby forcing the prefilled drug from the first drug chamber to the second drug chamber via at least one liquid passage connecting said two drug chambers,
ii. applying a force profile in a second direction on said at least partly rigid plunger, and via the plunger further to the liquid drug now contained in the second drug chamber, whereby the liquid drug is expelled from the second drug chamber through an expelling opening.

15. Method according to claim 14 where the expelling opening is an orifice.

16. Method according to claim 15 further comprising an intermediate step of removing a closure integrity member from the orifice and the area around the orifice immediate prior to step two, the closure integrity member being adapted to seal off the orifice and protect the area around the orifice.
